(19) Europäisches Patentamt
European Patent Office
Office européen des brevets

(11) EP 1 629 864 A1

(12) DEMANDE DE BREVET EUROPEEN

(43) Date de publication:
**01.03.2006 Bulletin 2006/09**

(51) Int Cl.:
***A61Q 5/06*** (2006.01) ***A61Q 5/10*** (2006.01)
***A61K 8/58*** (2006.01) ***A61K 8/49*** (2006.01)

(21) Numéro de dépôt: **05291518.8**

(22) Date de dépôt: **13.07.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **19.08.2004 FR 0451873**

(71) Demandeur: **L'OREAL**
**75008 Paris (FR)**

(72) Inventeur: **Plos, Grégory Gluck Heim Yoga**
**Tokyo 158-0097 (JP)**

(74) Mandataire: **Fevrier, Murielle Françoise E.**
**L'OREAL - D.I.P.I.**
**25-29 Quai Aulagnier**
**92600 Asnières (FR)**

# (54) Composition pour la teinture des fibres kératiniques comprenant un colorant direct méthinique contenant un motif benzoselenazolium

(57) La présente invention a pour objet une composition pour la teinture des fibres kératiniques comprenant, dans un milieu approprié pour la teinture, au moins un colorant direct méthinique contenant un motif benzosélènazolium, le procédé de teinture mettant en oeuvre cette composition, ainsi que l'utilisation pour la teinture des fibres kératiniques d'un colorant direct méthinique contenant un motif benzosélènazolium.

La présente invention permet d'obtenir une coloration des fibres kératiniques très chromatique, qui est visible sur cheveux foncés même sans traitement oxydant.

EP 1 629 864 A1

Printed by Jouve, 75001 PARIS (FR)

## Description

La présente invention a pour objet une composition pour la teinture des fibres kératiniques comprenant, dans un milieu approprié pour la teinture, au moins un colorant direct méthinique contenant un motif benzosélènazolium.

Il est connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer par diffusion à l'intérieur du cheveu, puis à rincer les fibres.

Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations temporaires ou semi-permanentes du fait de la nature des liaisons entre les colorants directs et la fibre kératinique. Ces interactions font que la désorption des colorants de la surface et / ou du coeur de la fibre se fait facilement. Les colorations présentent généralement une faible puissance tinctoriale et une faible tenue aux lavages et à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et / ou sur la fibre kératinique.

Ce type de coloration présente l'inconvénient de n'être généralement pas très visible sur cheveu foncé car le fond du cheveu masque la couleur du colorant qui a diffusé à l'intérieur.

Par contre, puisque l'application de la coloration se fait dans des conditions douces, c'est-à-dire sans agents oxydants, le cheveu n'est pas abîmé.

Le but de la présente invention est de fournir des compositions pour la teinture des fibres kératiniques à base de colorants directs qui ne présentent pas les inconvénients des compositions connues de l'art antérieur, en particulier des compositions pour la teinture des fibres kératiniques qui permettent d'obtenir des colorations visibles sur cheveux foncés même sans traitement oxydant.

Ce but est atteint avec la présente invention qui a pour objet une composition pour la teinture des fibres kératiniques comprenant, dans un milieu approprié pour la teinture, au moins un colorant direct méthinique contenant un motif benzosélènazolium.

La présente invention a également pour objet un procédé de teinture des fibres kératiniques mettant en oeuvre la composition conforme à l'invention.

La présente invention a aussi pour objet l'utilisation pour la teinture des fibres kératiniques d'un colorant direct méthinique contenant un motif benzosélènazolium.

La présente invention permet d'obtenir une coloration des fibres kératiniques très chromatique, qui est visible sur cheveux foncés même sans traitement oxydant.

Le ou les colorants directs méthiniques contenant un motif benzosélènazolium présents dans la composition conforme à l'invention sont choisis parmi les composés de formules (I) ou (II) suivantes :

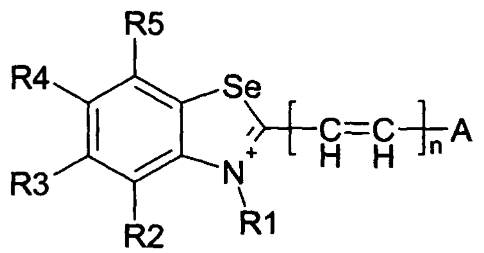

(I)

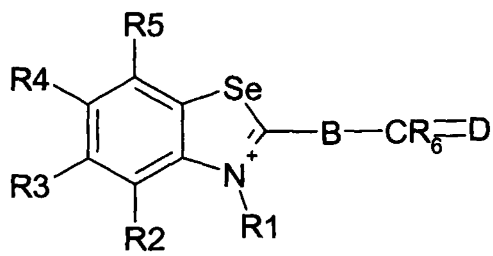

(II)

dans lesquelles :

- **$R_1$ à $R_5$** représentent, indépendamment les uns des autres, un atome d'hydrogène ; un atome d'halogène ; un radical hydroxyle ; un radical alcoxy ; un radical alkyle ; un radical amino ; un radical (alkyl)amino ; un radical di (alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl)amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl)aminoalkyle ; un radical di(hydroxyalkyl)aminoalkyle ; un radical (aminoalkyl)aminoalkyle ; un radical di(aminoalkyl)aminoalkyle ; un radical (alkyl)(hydroxyalkyl)aminoalkyle ; un radical (aminoalkyl)(alkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical alkyle substitué par un groupement ammonium quaternaire ; un radical carboxy ; un radical carboxyalkyle ; un radical alkyle substitué par un groupement sulfonate sous forme acide ou sous forme de sel ; un radical alkylcarbonylalkyle ; deux groupements adjacents pouvant former ensemble et avec les atomes sur lesquels ils sont fixés un cycle aromatique condensé, substitué ou non ;
- **n** représente un nombre entier de 1 à 4 ;
- **A** représente un groupement choisi parmi les groupements suivants :

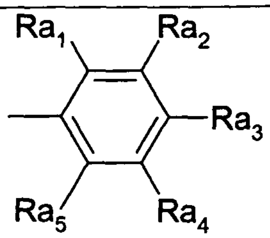

A1

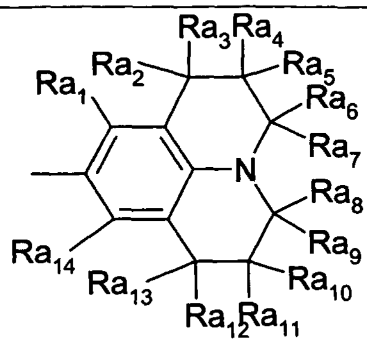

A2

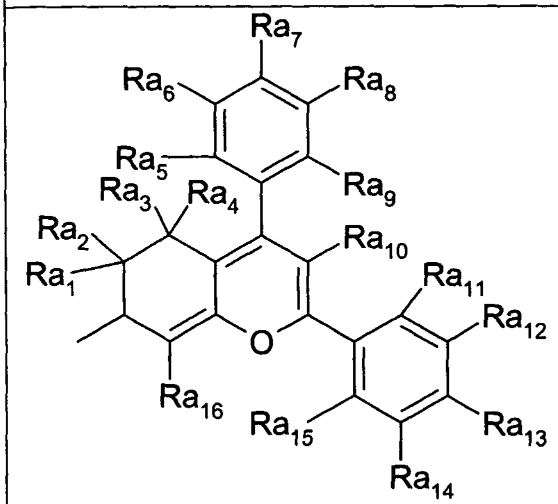

A3

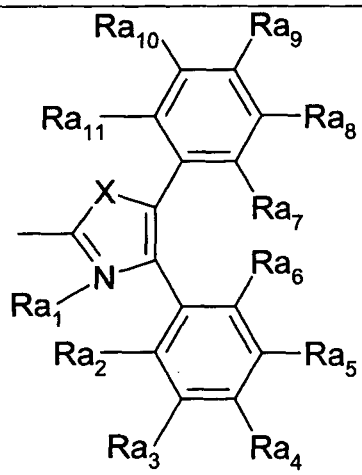

A4

tels que :

- **X** représente un atome d'oxygène, un atome de soufre, un atome de sélénium, un groupement $CR_aR_b$ ou un groupement $NR_a$, avec $R_a$ et $R_b$ représentant, indépendamment l'un de l'autre, un atome d'hydrogène ou un radical alkyle ;
- **$Ra_1$** à **$Ra_{16}$** représentent, indépendamment les uns des autres, un atome d'hydrogène ; un atome d'halogène ; un radical hydroxyle ; un racical alcoxy ; un radical alkyle ; un radical amino ; un radical (alkyl)amino ; un radical di(alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl) amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl)aminoalkyle ; un radical di (hydroxyalkyl)aminoalkyle ; un radical (aminoalkyl)aminoalkyle ; un radical di(aminoalkyl)aminoalkyle ; un radical (alkyl)(hydroxyalkyl)aminoalkyle ; un radical (aminoalkyl)(alkyl)amino ; un radical (aminoalkyl)(hydroxyalkyl)aminoalkyle ; un radical phényle éventuellement substitué ; un radical phénylalkyle éventuellement substitué; un groupement ammonium quaternaire ; un radical carboxy ; un groupement carboxyalkyle ; un radical alkyle substitué par un groupement ammonium quaternaire ; deux groupements adjacents pouvant former ensemble et avec les atomes sur lesquels ils sont fixés un cycle aromatique condensé, substitué ou non ;

- **B** représente un groupement choisi parmi les groupements suivants :

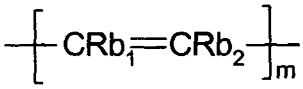
B1

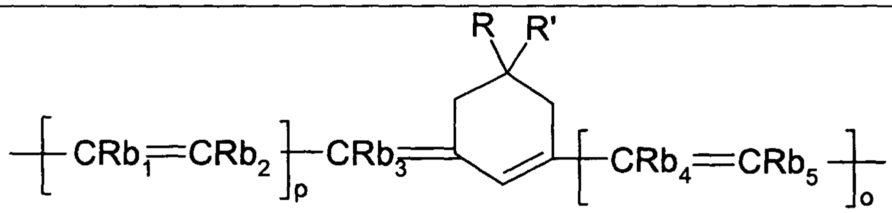
B2

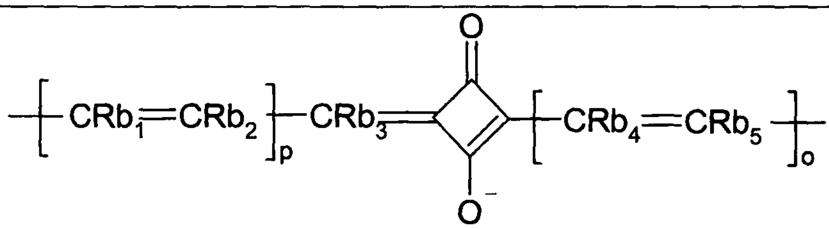
B3

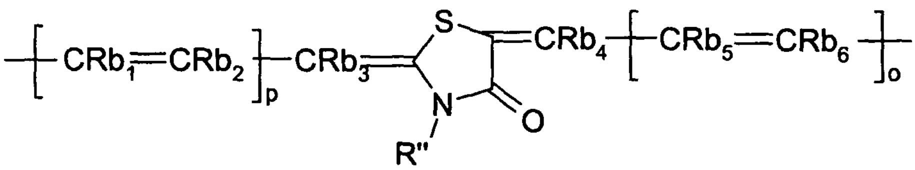
B4

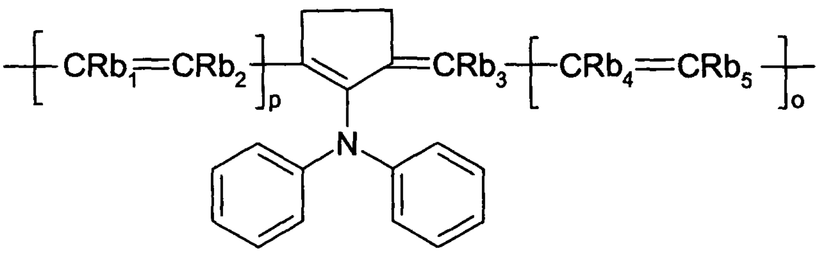
B5

tels que :

- **m** représente un nombre entier de 0 à 3 ;
- **o+p** représente un nombre entier de 0 à 1 ;

- **$Rb_1$, $Rb_2$, $Rb_3$, $Rb_4$, $Rb_5$, $Rb_6$** représentent, indépendamment les uns des autres, un atome d'hydrogène ; un atome d'halogène ; un radical alkyle en $C_1$-$C_3$ ; un radical phényle ;
- **R, R' et R"** représentent indépendamment les uns des autres, un atome d'hydrogène ; un atome d'halogène ou un radical alkyle ;

- **$R_6$** représente un atome d'hydrogène ; un atome d'halogène ; un radical alkyle en $C_1$-$C_3$; un radical phényle ;
- **D** représente un groupement choisi parmi les groupements suivants :

| | |
|---|---|
| 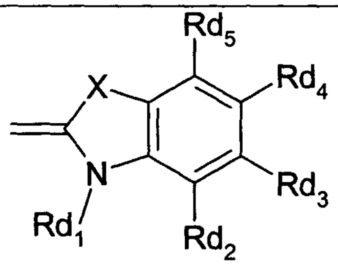 D1 | 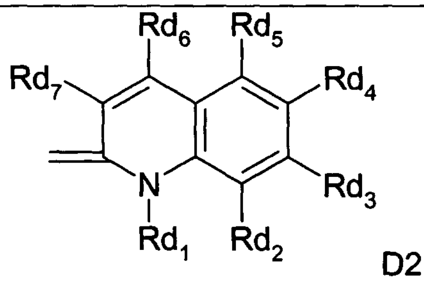 D2 |
| 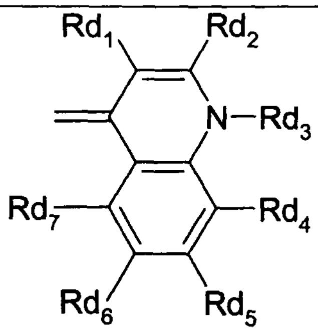 D3 | 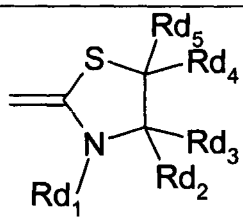 D4 |

| | |
|---|---|
| 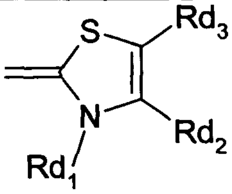 D5 | 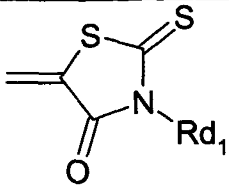 D6 |
| 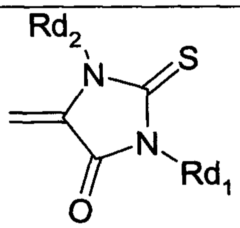 D7 | 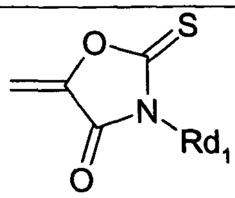 D8 |
| 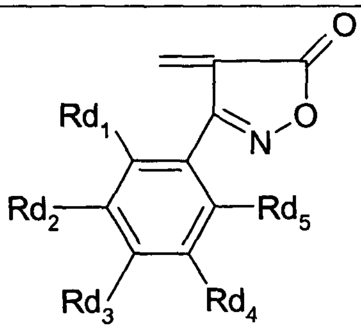 D9 | 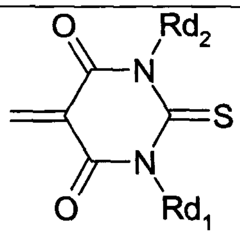 D10 |
| 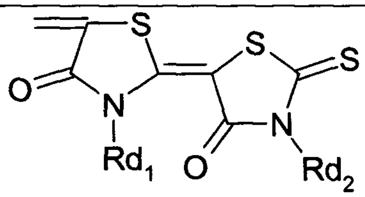 D11 | 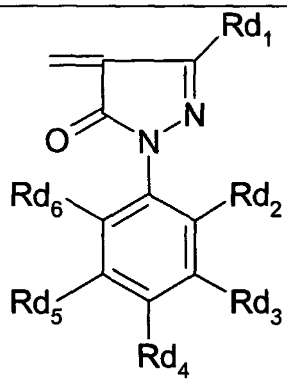 D12 |
| 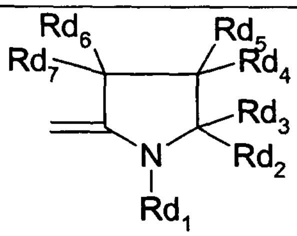 D13 | 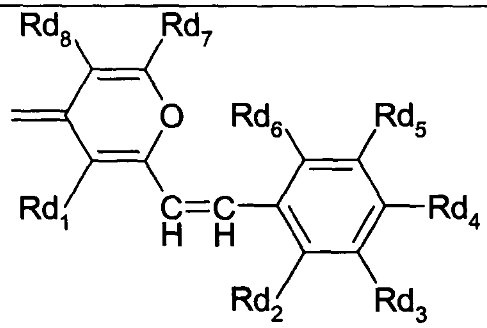 D14 |

| | |
|---|---|
| 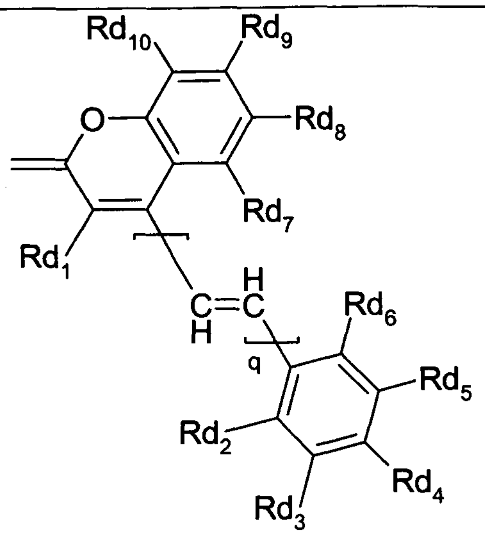 D15 | |

tels que :

- **X** représente un atome d'oxygène, un atome de soufre, un atome de sélénium, un groupement $CR_cR_d$ ou $NR_c$, avec $R_c$ et $R_d$ représentent, indépendamment l'un de l'autre, un atome d'hydrogène ou un radical alkyle ;
- **q** représente un nombre entier égal à 1 ou 2 ;
- $\mathbf{Rd_1}$ à $\mathbf{Rd_{10}}$ représentent, indépendamment les uns des autres, un atome d'hydrogène ; un atome d'halogène ; un radical hydroxyle ; un radical alcoxy ; un radical alkyle ; un radical amino ; un radical (alkyl)amino ; un radical di(alkyl)amino ; un radical (hydroxyalkyl)amino ; un radical di(hydroxyalkyl)amino ; un radical (aminoalkyl) amino ; un radical di(aminoalkyl)amino ; un radical (alkyl)(hydroxyalkyl)amino ; un radical (alkyl)(aminoalkyl) amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl)aminoalkyle ; un radical di (hydroxyalkyl)aminoalkyle ; un radical (aminoalkyl)aminoalkyle ; un radical di(aminoalkyl)aminoalkyle ; un radical (alkyl)(hydroxyalkyl)aminoalkyle ; un radical (alkyl)(aminoalkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical carboxy; un radical carboxyalkyle; un radical alkényle ; un groupement sulfonate sous forme acide ou sous forme de sel ; un radical alkyle substitué par un groupement sulfonate sous forme acide ou sous forme de sel ; un radical alkyle substitué par un groupement ammonium quaternaire ; deux groupements adjacents pouvant former ensemble et avec les atomes sur lesquels ils sont fixés un cycle aromatique condensé, substitué ou non.

Dans les définitions ci-dessus, les radicaux alkyle sont linéaires ou ramifiés, cycliques ou acycliques, et comprennent, sauf indication contraire, de 1 à 5 atomes de carbone, par exemple un radical méthyle, éthyle, n-propyle, iso-propyle, n-butyle, ter-butyle, cyclopentyle.

Un radical alcoxy est un radical alk-O-, un radical mono ou dialkylamino est un radical $-N(alk)_n$ avec n = 1 ou 2, un radical alkylcarbonylalkyle est un radical alk-CO-alk-, dans chacune de ces définitions le radical alkyle étant tel que défini précédemment.

Un radical alkényle est un radical hydrocarboné comprenant de 2 à 20 atomes de carbone et une ou plusieurs doubles liaisons, par exemple un radical vinyle, propènyle, butènyle.

Un radical alkyle substitué est un radical mono ou polysubstitué. Par exemple, un hydroxyalkyle, un aminoalkyle, un carboxyalkyle ou un phénylalkyle est un radical alkyle qui peut être substitué par un ou plusieurs radicaux hydroxy, amino, carboxy ou phényle.

Un radical phényle peut être substitué par un ou plusieurs substituants choisis parmi un atome d'halogène, un radical hydroxyle, un radical amino, un radical alkylamino, un radical dialkylamino, un radical alkyle, un radical alcoxy, un groupement sulfonate sous forme acide ou sous forme de sel, un groupement $-NH_3^+$.

On entend par cycle aromatique condensé un cycle aromatique comprenant de 5 à 11 chaînons, un ou plusieurs atomes de carbone pouvant être remplacés par un atome d'oxygène, d'azote, de soufre ou de phosphore, par exemple

un cycle benzène, pyridine, thiophène, pyrimidine, anthracène, qui est accolé à un deuxième cycle avec lequel il présente au moins deux atomes communs. Ce cycle aromatique peut être substitué par un ou plusieurs substituants choisis parmi un atome d'halogène, un radical hydroxyle, un radical amino, un radical alkylamino, un radical dialkylamino, un radical alkyle, un radical alcoxy, un groupement sulfonate sous forme acide ou sous forme de sel, un groupement $-NH_3^+$.

Parmi les groupements ammonium quaternaire, on peut par exemple citer le groupement $-NH_3^+$, le groupement pyridinium, le groupement imidazolium, le groupement triazolium et le groupement pyrazolium.

Selon un mode de réalisation particulier de l'invention, $R_1$ à $R_5$ représentent, indépendamment les uns des autres, un atome d'halogène ; un radical hydroxyle ; un radical alcoxy ; un radical alkyle ; un radical amino ; un radical (alkyl)amino ; un radical di(alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl) amino ; un radical (hydroxyalkyl)(aminoalkyl)amino ; un radical hydroxyalkyle ; un radical aminoalkyle ; un radical (alkyl) aminoalkyle ; un radical di(alkyl)aminoalkyle ; un radical (hydroxyalkyl)aminoalkyle ; un radical di(hydroxyalkyl) aminoalkyle ; un radical (aminoalkyl)aminoalkyle ; un radical di(aminoalkyl)aminoalkyle ; un radical (alkyl)(hydroxyalkyl) aminoalkyle ; un radical (aminoalkyl)(alkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical alkyle substitué par un groupement ammonium quaternaire ; un radical carboxy ; un radical carboxyalkyle ; un radical alkyle substitué par un groupement sulfonate sous forme acide ou sous forme de sel ; un radical alkylcarbonylalkyle ; deux groupements adjacents pouvant former ensemble et avec les atomes sur lesquels ils sont fixés un cycle aromatique condensé, substitué ou non.

Le ou les colorants directs méthiniques contenant un motif benzosélènazolium de formules (I) ou (II) peuvent, le cas échéant, être associés à un contre-ion négatif. De préférence, le contre-ion négatif représente un halogénure, un perchlorate, un p-toluène-sulfonate, un m-toluène-sulfonate, un o-toluène-sulfonate, un térafluoroborate, un alkyl $(C_1\text{-}C_5)$sulfate ou un sulfonate.

Selon un mode de réalisation particulier de l'invention, $R_1$ à $R_5$ sont choisis parmi un atome d'hydrogène ; un radical alkyle ; un radical alkyle substitué par un groupement sulfonate sous forme acide ou sous forme de sel. Dans ce cas, $R_1$ à $R_5$ sont par exemple un atome d'hydrogène ; un radical méthyle ; un radical éthyle ; un radical n-propyle substitué par un groupement sulfonate sous forme de sel.

Selon un mode de réalisation particulier de l'invention, n est égal à 1.

Selon un mode de réalisation particulier de l'invention, A représente un groupement A1. Selon un mode de réalisation préféré, $Ra_1$ à $Ra_5$ sont choisis parmi un atome d'hydrogène ; un radical di(alkyl)amino. Dans ce cas, $Ra_1$ à $Ra_5$ sont par exemple un atome d'hydrogène ; un radical diéthylamino ; un radical diméthylamino ; un radical méthyléthylamino.

Selon un mode de réalisation particulier de l'invention, B représente un groupement B1. Selon un mode de réalisation préféré, $Rb_1$ et $Rb_2$ sont choisis parmi un atome d'hydrogène ; un radical alkyle. Dans ce cas, $Rb_1$ et $Rb_2$ sont par exemple un atome d'hydrogène ; un radical méthyle ; un radical éthyle.

Selon un mode de réalisation particulier de l'invention, $R_6$ est choisi parmi un atome d'hydrogène.

Selon un mode de réalisation particulier de l'invention, D représente un groupement D1 ou un groupement D2. Selon un mode de réalisation préféré, X représente un atome de sélénium. Selon un autre mode de réalisation préféré, $Rd_1$ à $Rd_7$ sont choisis parmi un atome d'hydrogène ; un radical alkyle ; un radical alkyle substitué par un groupement sulfonate sous forme acide ou sous forme de sel. Dans ce cas, $Rd_1$ à $Rd_7$ sont par exemple un atome d'hydrogène ; un radical méthyle ; un radical éthyle ; un radical n-propyle substitué par un groupement sulfonate sous forme acide.

Selon un mode de réalisation préféré de l'invention, le ou les colorants directs méthiniques contenant un motif benzosélènazolium sont choisis parmi les composés de formules (Ia), (IIa) ou (IIb) suivantes :

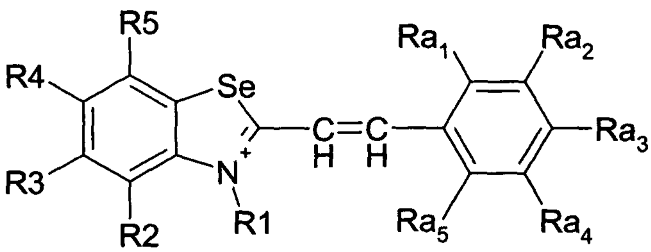

(Ia)

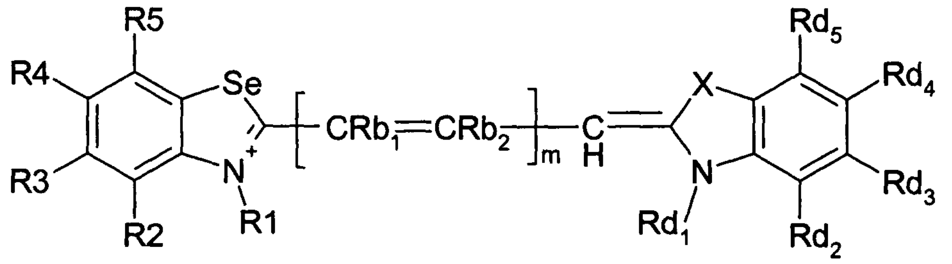

(IIa)

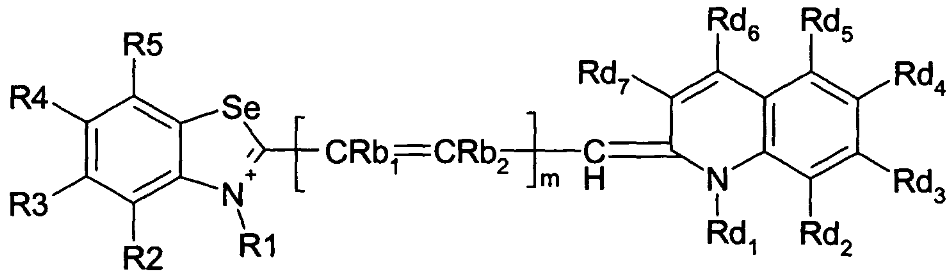

(IIb)

dans lesquelles :

- **$R_1$** à **$R_5$** représentent, indépendamment les uns des autres, un atome d'hydrogène ; un radical alkyle ; un radical alkyle substitué par un groupement sulfonate sous forme acide ou sous forme de sel ;
- **$Ra_1$** à **$Ra_5$** représentent, indépendamment les uns des autres, un atome d'hydrogène ; un radical di(alkyl)amino ;
- m représente un nombre entier de 0 à 3 ;
- **$Rb_1$** et **$Rb_2$** représentent, indépendamment les uns des autres, un atome d'hydrogène ; un radical alkyle en $C_1$-$C_3$ ;
- **$Rd_1$** à **$Rd_7$** représentent, indépendamment les uns des autres, un atome d'hydrogène ; un radical alkyle ; un radical alkyle substitué par un groupement sulfonate sous forme acide ou sous forme de sel ;
- **X** représente un atome d'oxygène, un atome de soufre, un atome de sélénium, un groupement $CR_cR_d$ ou $NR_c$, avec $R_c$ et $R_d$ représentent, indépendamment l'un de l'autre, un atome d'hydrogène ou un radical alkyle.

De préférence, X représente un atome de sélénium.

A titre d'exemples de colorants directs méthiniques contenant un motif benzosélènazolium de formules (I) ou (II), on peut citer les colorants suivants :

| | |
|---|---|
| Colorant 1 | 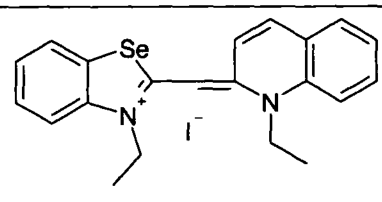 |
| Colorant 2 | 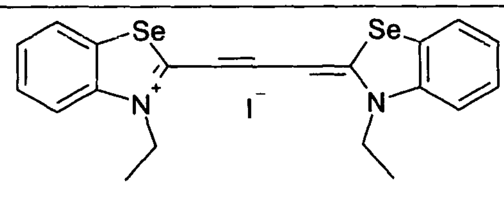 |

| | |
|---|---|
| Colorant 3 | 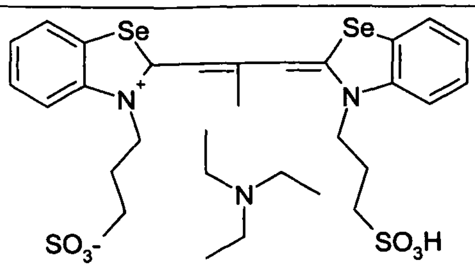 |
| Colorant 4 | 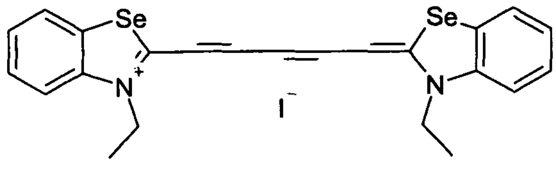 |
| Colorant 5 | 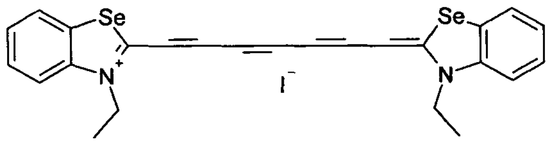 |
| Colorant 6 | 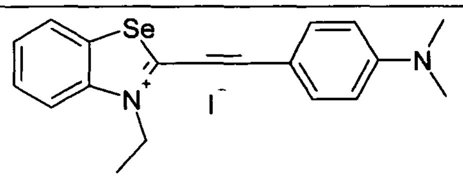 |
| Colorant 7 | 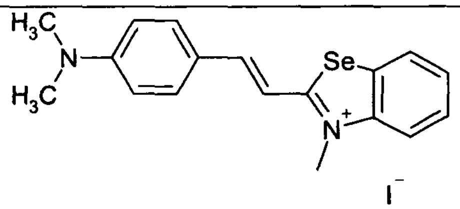 |

Les colorants directs méthiniques contenant un motif benzosélènazolium utiles dans le cadre de l'invention sont des composés connus de la technique. Ils peuvent par exemple être synthétisés selon le mode de préparation décrit dans la publication New fluorescent stains for protein detection in sodium dodecyl sulfate-polyacrylamide gels, Soo Yeon Hong, Hyunsook Jun, Seung Soo Yoon, Chulhun Kang, Myungkoo Suh, Chemistry Letters, vol. 33, N°3 (2004).

Le ou les colorants directs méthiniques contenant un motif benzosélènazolium utiles dans le cadre de l'invention sont chacun généralement présents en quantité comprise entre 0,0001 et 30 % en poids par rapport au poids total de la composition, de préférence entre 0,001 et 20 % en poids par rapport au poids total de la composition, et encore plus préférentiellement entre 0,01 et 15 % en poids par rapport au poids total de la composition.

La composition conforme à l'invention peut en outre comprendre un ou plusieurs colorants directs additionnels différents des colorants directs méthiniques contenant un motif benzosélènazolium.

A titre d'exemples, ces colorants directs additionnels sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthytamino)-2-nitro-benzène, le 1-β-hydroxyéthylamino-2-nitro-4-bis-(β-hydroxyéthylamino)-benzène, le 1-β-hydroxyéthylamino-2-nitro-4-amino-benzène, le 1-β-hydroxyéthytamino-2-nitro-4-(éthyl)(β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitrobenzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitro-benzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitrobenzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772 et EP 0 714 954 dont le contenu fait partie intégrante de l'invention.

Parmi ces composés, on peut tout particulièrement citer le chlorure de 1,3-diméthyl-2-[[4-(diméthylamino) phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3$^{e}$ édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Lorsque la composition conforme à l'invention comprend un ou plusieurs colorants directs additionnels, ils sont chacun généralement présents en quantité comprise entre 0,0001 et 30 % en poids par rapport au poids total de la composition, de préférence entre 0,001 et 20 % en poids par rapport au poids total de la composition, et encore plus préférentiellement entre 0,01 et 15 % en poids par rapport au poids total de la composition.

La composition conforme à l'invention peut en outre comprendre une ou plusieurs bases d'oxydation conventionnellement utilisées pour la teinture des fibres kératiniques.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyt)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N, N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo [1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsque la composition conforme à l'invention comprend une ou plusieurs bases d'oxydation, elles sont chacune généralement présentes en quantité comprise entre 0,001 et 10 % en poids environ par rapport au poids total de la composition, de préférence entre 0,005 et 6 % en poids environ.

Lorsque la composition conforme à l'invention comprend une ou plusieurs bases d'oxydation, elle peut en outre comprendre un ou plusieurs coupleurs conventionellement utilisés pour la teinture des fibres kératiniques.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Lorsque la composition conforme à l'invention comprend un ou plusieurs coupleurs, ils sont chacun généralement présents en quantité comprise entre 0,001 et 10 % en poids environ par rapport au poids total de la composition, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther de diéthylèneglycol ; ainsi que les alcools aromatiques comme l'alcool benzylique ou le phénoxyéthanol, les produits analogues et leurs mélanges.

Les solvants peuvent être présents dans des proportions de préférence comprises entre 1 et 40% en poids par rapport au poids total de la composition tinctoriale, et plus préférentiellement encore entre 5 et 30% en poids environ.

La composition conforme à la présente invention peut également renfermer divers adjuvants utilisés classiquement dans les compositions pour la teinture des fibres kératiniques, tels que des agents tensioactifs anioniques, cationiques, non ioniques, amphotères, zwitterioniques ou leurs mélanges, des polymères anioniques, cationiques, non ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des agents dispersants, des agents de conditionnement tels que par exemple des silicones volatiles ou non volatiles, modifiées ou non modifiées, des chitosanes et leurs dérivés, des agents filmogènes, des céramides, des agents conservateurs, des agents opacifiants, des acides aminées, des enzymes.

Les adjuvants ci-dessus sont chacun généralement présents en quantité comprise entre 0,01 et 20 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soit pas, ou substantiellement pas, altérées par la ou les adjonctions envisagées.

Le pH de la composition conforme à l'invention est généralement compris entre 2 et 12 environ, et de préférence entre 3 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide étidronique, l'acide chlorhydrique, l'acide orthophosphorique, l'acide sulfurique, les acides carboxyliques comme l'acide acétique, l'acide tartrique, l'acide citrique, l'acide lactique, et les acides sulfoniques.

Parmi les agents alcalinisants, on peut citer, à titre d'exemple, l'ammoniaque, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (III) suivante :

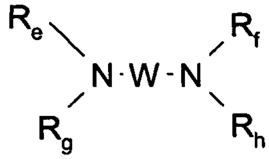

(III)

dans laquelle W est un reste propylène éventuellement substitué par un groupement hydroxyle ou un radical alkyle en $C_1$-$C_4$ ; $R_e$, $R_f$, $R_g$ et $R_h$, identiques ou différents, représentent un atome d'hydrogène, un radical alkyle en $C_1$-$C_4$ ou hydroxyalkyle en $C_1$-$C_4$.

La composition conforme à l'invention peut se présenter sous des formes diverses, telles que sous forme de solutions, d'émulsions, de crèmes, de gels, éventuellement pressurisés sous forme de mousses, ou sous toute autre forme appropriée pour réaliser une teinture des fibres kératiniques.

La présente invention a également pour objet un procédé de teinture des fibres kératiniques, caractérisé en ce que l'on applique sur les fibres kératiniques une composition de teinture conforme à l'invention telle que définie précédemment pendant un temps de pose suffisant pour obtenir la coloration désirée.

La composition conforme à l'invention peut être appliquée sur tout type de cheveux, tels que des cheveux blancs ou clairs, ainsi que sur des cheveux foncés, naturellement ou artificiellement.

Le temps de pose est généralement compris entre 5 minutes et 1 heure, de préférence entre 15 minutes et 1 heure.

La température d'application est généralement fixée entre la température ambiante et 80°C, de préférence entre la température ambiante et 60 °C.

Lorsque la composition conforme à l'invention comprend au moins une base d'oxydation et éventuellement au moins un coupleur, ou si l'on désire réaliser une coloration directe éclaircissante, on peut utiliser un agent oxydant. Cet agent oxydant peut être ajouté à la composition de l'invention juste au moment de l'emploi ou il peut être mis en oeuvre à partir d'une composition oxydante le contenant, appliquée simultanément
ou séquentiellement à la composition de l'invention.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et les persulfates, les peracides et les enzymes oxydases parmi lesquelles on peut citer les peroxydases, les oxydo-réductases à deux électrons telles que les uricases et les oxygénases à quatre électrons comme les laccases. Le peroxyde d'hydrogène est particulièrement préféré.

La présente invention a également pour objet l'utilisation pour la teinture des fibres kératiniques d'un colorant direct méthinique contenant un motif benzosélènazolium.

Selon un mode de réalisation particulier de l'invention, le ou les colorants directs méthiniques contenant un motif benzosélènazolium sont choisis parmi les composés de formules (I) ou (II) telles que définies précédemment.

Les exemples qui suivent servent à illustrer l'invention sans toutefois présenter un caractère limitatif.

## EXEMPLES

Exemples 1 à 6 :

Les compositions tinctoriales suivantes ont été préparées :

| **Composition** | **1** | **2** | **3** | **4** | **5** | **6** |
|---|---|---|---|---|---|---|
| Colorant 1 | $3.10^{-3}$ mole | - | - | - | - | - |
| Colorant 2 | - | $3.10^{-3}$ mole | - | - | - | - |
| Colorant 3 | - | - | $3.10^{-3}$ mole | - | - | - |
| Colorant 4 | - | - | - | $3.10^{-3}$ mole | - | - |
| Colorant 5 | - | - | - | - | $3.10^{-3}$ mole | - |
| Colorant 6 | - | - | - | - | - | $3.10^{-3}$ mole |
| Support de teinture commun | (*) | (*) | (*) | (*) | (*) | (*) |

Suite de tableau

| Composition | 1 | 2 | 3 | 4 | 5 | 6 |
|---|---|---|---|---|---|---|
| Eau déminéralisée q.s.p. | 100 g | 100 g | 100 g | 100 g | 100 g | 100 g |
| (*) : Support de teinture commun | | | | | | |

| | |
|---|---|
| Hydroxyéthyl cellulose vendue par la société Aqualon sous la dénomination Natrosol 250 MR | 0,384 g |
| Mélange de p-hydroxybenzoates de méthyle, butyle, éthyle, propyle et isobutyle vendu par la société NIPA sous la dénomination NIPA ester 82121 | 0,032 g |
| Alkyl($C_8$/$C_{10}$ 50/50)polyglucoside vendu par la société SEPPIC sous la dénomination Oramix CG110 | 5 g |
| Alcool benzylique | 4 g |
| Polyéthylène glycol (8 OE) | 6 g |

Les structures des colorants 1 à 6 sont telles que définies précédemment.
Les compositions sont appliquées sur des mèches de cheveux gris à 90 % de blancs naturels et permanentés, à raison de 5 g de composition pour 1 g de cheveux. La température d'application est de 60 °C et le temps de pose d'une heure. Les cheveux sont ensuite rincés et séchés au casque pendant 30 minutes.

Exemples 7 à 14 :

Les compositions tinctoriales suivantes ont été préparées :

| Composition | 7 | 8 | 9 | 10 | 11 | 12 | 13 | 14 |
|---|---|---|---|---|---|---|---|---|
| Colorant 7 | $3.10^{-3}$ mole | $3.10^{-3}$ mole | $3.10^{-3}$ mole | $3.10^{-3}$ mole | $3.10^{-3}$ mole | $3.10^{-3}$ mole | $3.10^{-3}$ mole | $3.10^{-3}$ mole |
| Support de teinture A | q.s.p. 50 g | q.s.p. 50 g | q.s.p. 50 g | q.s.p. 50 g | - | - | - | - |
| Support de teinture B | - | - | - | - | q.s.p. 50 g | q.s.p. 50 g | q.s.p. 50 g | q.s.p. 50 g |
| Eau distillée | 50 g | - | - | - | 50 g | - | - | - |
| Tampon citrate pH = 4 500 mM | - | 50 g | - | - | - | 50 g | - | - |
| Tampon phosphate pH = 7 500 mM | - | - | 50 g | - | - | - | 50 g | - |
| Tampon borate pH = 9 500 mM | - | - | - | 50 g | - | - | - | 50 g |

Support de teinture A :

| | |
|---|---|
| Polyéthylène glycol (8 OE) | 12 g |
| Mélange p-hydroxybenzoates de méthyle, butyle, éthyle, propyle, isobutyle (7/57/22/14) | 0,12 g |
| Eau désionisée | 68,44 g |
| Hydroxyéthyl cellulose | 1,44 g |
| Alkyl ($C_8$/$C_{10}$ 50/50) polyglucoside en solution aqueuse tamponnée à pH 5 | 10 g |

Suite de tableau

| | |
|---|---|
| Alcool benzylique | 8 g |

Support de teinture B :

| | |
|---|---|
| Ethanol | 30 g |
| Alcool benzylique | 10 g |
| Acide benzoïque | 0,4 g |
| Hydroxyéthyl cellulose | 1,44 g |
| Eau distillée | q.s.p. 100 g |

La structure du colorant 7 est telle que définie précédemment.
Les compositions sont appliquées sur des mèches de cheveux gris à 90 % de blancs naturels et permanentés, à raison de 5 g de composition pour 1 g de cheveux, pendant 30 minutes à température ambiante.
A l'issue de la coloration, les mèches sont simplement rincées à l'eau claire et séchées à 60 °C.
La couleur des mèches est mesurée au spectrocolorimètre CM3600d (composantes spéculaires inclues, angle 10°, illuminant D65) dans le système CIEL*a*b*. Dans ce système, L* représente l'intensité de la couleur, a* indique l'axe de couleur vert / rouge et b* l'axe de couleur bleu / jaune.
ΔE représente la variation de couleur entre une mèche de cheveux non colorée et une mèche de cheveux colorée et est déterminé à partir de la formule suivante :

$$\Delta E = \sqrt{(L^*-L_o^*)^2 + (a^*-a_o^*)^2 + (b^*-b_o^*)^2}$$

dans laquelle L*, a* et b* représentent les valeurs mesurées sur la mèche colorée et $L_0^*$, $a_0^*$ et $b_0^*$ représentent les valeurs mesurées sur la mèche non colorée.
Les résultats colorimétriques obtenus sont donnés dans les tableaux ci-après.

| Mèche à 90 % de cheveux blancs naturels | | | | |
|---|---|---|---|---|
| | L* | a* | b* | ΔE |
| Composition 7 | 40,8 | 31,3 | - 1,8 | 43 |
| Composition 8 | 43,2 | 27,4 | - 1,6 | 38 |
| Composition 9 | 37,5 | 36,2 | - 3,9 | 49 |
| Composition 10 | 36,5 | 35,6 | 0,9 | 48 |
| Composition 11 | 32,2 | 31,2 | 1,1 | 47 |
| Composition 12 | 27,1 | 27,1 | 0,8 | 49 |
| Composition 13 | 29,6 | 27,2 | 0,1 | 47 |
| Composition 14 | 36,3 | 26,6 | 0,7 | 41 |

| Mèche à 90 % de cheveux blancs permanentés | | | | |
|---|---|---|---|---|
| | L* | a* | b* | ΔE |
| Composition 7 | 42,1 | 44,8 | - 4,1 | 51 |
| Composition 8 | 40,5 | 38,5 | 1,1 | 44 |

Suite de tableau

| Mèche à 90 % de cheveux blancs permanentés | | | | |
|---|---|---|---|---|
| | L* | a* | b* | ΔE |
| Composition 9 | 43,6 | 41,4 | - 4,3 | 47 |
| Composition 10 | 31,8 | 40,0 | - 2,1 | 51 |
| Composition 11 | 34,9 | 42,9 | - 5,1 | 52 |
| Composition 12 | 30,8 | 40,3 | - 3,8 | 52 |
| Composition 13 | 37,5 | 41,1 | - 4,8 | 50 |
| Composition 14 | 37,5 | 40,5 | - 4,9 | 49 |

Un test de ténacité aux shampooings a été réalisé sur les mèches de cheveux colorés avec la composition 11 décrite ci-dessus. Les shampooings ont été réalisés manuellement.

La dégradation de la couleur après x shampooings est estimée selon la formule suivante :

$$\%dégradation = 100 * \frac{DE\ xShamp}{DE\ Col}$$

avec :

$$DE\ Col = \sqrt{(a*col - a*tém)^2 + (b*col - b*tém)^2 + (L*col - L*tém)^2}$$

et

$$DE\ xShamp = \sqrt{(a*col - a*xSh)^2 + (b*col - b*xSh)^2 + (L*col - L*xSh)^2}$$

dans lesquelles $a^*_{tém}$, $b^*_{tém}$ et $L^*_{tém}$ sont les valeurs de a*, b* et L* de la mèche non colorée, $a^*_{col}$, $b^*_{col}$ et $L^*_{col}$ les valeurs de a*, b* et L* de la mèche colorée avant shampooing, tandis que $a^*_{xSh}$, $b^*_{xSh}$ et $L^*_{xSh}$ les valeurs de a*, b* et L* de la mèche colorée après x shampooings.

Les résultats colorimétriques obtenus sont donnés dans les tableaux ci-après.

| Mèche à 90 % de cheveux blancs naturels | | | | |
|---|---|---|---|---|
| | L* | a* | b* | % dégradation |
| 0 shampooing | 33,86 | 26,54 | 2,95 | - |
| 5 shampooings | 43,2 | 30,65 | 0,76 | 26 |

| Mèche à 90 % de cheveux blancs permanentés | | | | |
|---|---|---|---|---|
| | L* | a* | b* | % dégradation |
| 0 shampooing | 34,35 | 36,9 | - 0,91 | - |
| 5 shampooings | 40,98 | 39,87 | - 0,16 | 15 |

Ces résultats montrent qu'après 5 shampooings, les colorants utiles dans le cadre de l'invention présentent une bonne ténacité.

Les performances d'éclaircissement de la composition de l'exemple 9 sont exprimées en fonction de la réflectance des cheveux.

La réflectance, mesurée au moyen d'un spectrophotocolorimètre, est la quantité de lumière réémise par les cheveux après exposition avec une lumière visible dans la gamme de longueurs d'onde allant de 400 à 700 nanomètres. La figure 1 représente la réflectance en fonction de la longueur d'onde de cheveux blancs permanentés et de cheveux blancs permanentés et colorés avec la composition de l'exemple 9.
On constate que les cheveux colorés avec la composition de l'exemple 9 fluorescent, ce qui se traduit par un effet d'éclaircissement des cheveux.

## Revendications

1. Composition pour la teinture des fibres kératiniques comprenant, dans un milieu approprié pour la teinture, au moins un colorant direct méthinique contenant un motif benzosélènazolium choisi parmi les composés de formules (I) ou (II) suivantes :

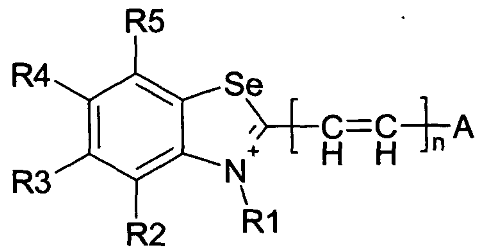

(I)

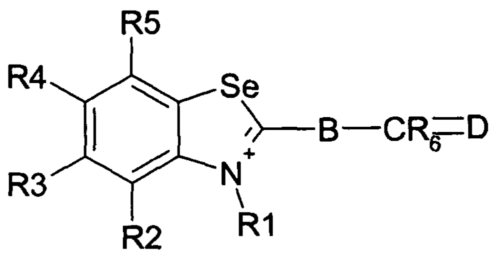

(II)

dans lesquelles :

• **$R_1$** à **$R_5$** représentent, indépendamment les uns des autres, un atome d'hydrogène ; un atome d'halogène ; un radical hydroxyle ; un radical alcoxy ; un radical alkyle ; un radical amino ; un radical (alkyl)amino ; un radical di(alkyl)amino ; un radical (hydroxyalkyl)amino ; un radical di(hydroxyalkyl)amino ; un radical (aminoalkyl)amino; un radical di(aminoalkyl)amino ; un radical (alkyl)(hydroxyalkyl)amino ; un radical (alkyl)(aminoalkyl)amino ; un radical (hydroxyalkyl)(aminoalkyl)amino ; un radical hydroxyalkyle ; un radical aminoalkyle ; un radical (alkyl) aminoalkyle ; un radical di(alkyl)aminoalkyle ; un radical (hydroxyalkyl)aminoalkyle ; un radical di(hydroxyalkyl) aminoalkyle ; un radical (aminoalkyl)aminoalkyle ; un radical di(aminoalkyl)aminoalkyle ; un radical (alkyl)(hydroxyalkyl)aminoalkyle ; un radical (aminoalkyl)(alkyl)amino ; un radical (aminoalkyl)(hydroxyalkyl) aminoalkyle ; un radical phényle éventuellement substitué ; un radical phénylalkyle éventuellement substitué ; un groupement ammonium quaternaire ; un radical alkyle substitué par un groupement ammonium quaternaire ; un radical carboxy ; un radical carboxyalkyle ; un radical alkyle substitué par un groupement sulfonate sous forme acide ou sous forme de sel ; un radical alkylcarbonylalkyle ; deux groupements adjacents pouvant former ensemble et avec les atomes sur lesquels ils sont fixés un cycle aromatique condensé, substitué ou non ;
• **n** représente un nombre entier de 1 à 4 ;
• **A** représente un groupement choisi parmi les groupements suivants :

| | |
|---|---|
| 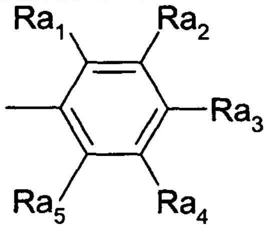 A1 | 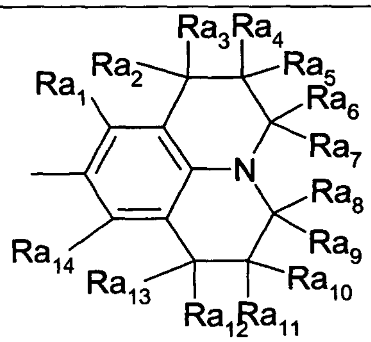 A2 |
| 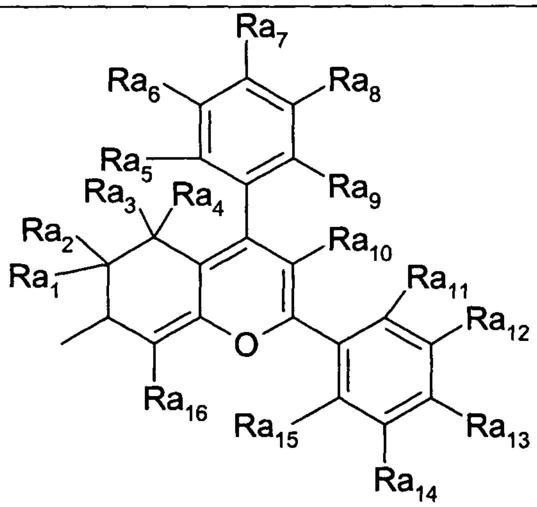 A3 | 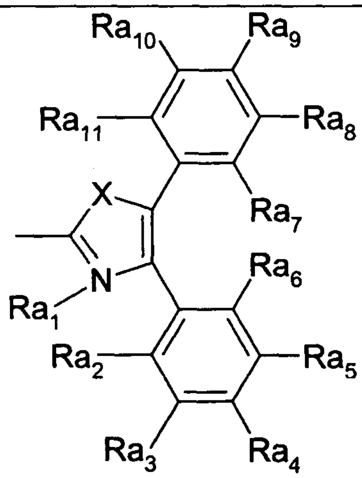 A4 |

tels que :

- **X** représente un atome d'oxygène, un atome de soufre, un atome de sélénium, un groupement $CR_aR_b$ ou un groupement $NR_a$, avec $R_a$ et $R_b$ représentant, indépendamment l'un de l'autre, un atome d'hydrogène ou un radical alkyle ;
- **$Ra_1$** à **$Ra_{16}$** représentent, indépendamment les uns des autres, un atome d'hydrogène ; un atome d'halogène ; un radical hydroxyle ; un racical alcoxy ; un radical alkyle ; un radical amino ; un radical (alkyl) amino ; un radical di(alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl)amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl) aminoalkyle ; un radical di(hydroxyalkyl)aminoalkyle ; un radical (aminoalkyl)aminoalkyle ; un radical di (aminoalkyl)aminoalkyle ; un radical (alkyl)(hydroxyalkyl)aminoalkyle ; un radical (aminoalkyl)(alkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical carboxy ; un groupement carboxyalkyle ; un radical alkyle substitué par un groupement ammonium quaternaire ; deux groupements adjacents pouvant former ensemble et avec les atomes sur lesquels ils sont fixés un cycle aromatique condensé, substitué ou non ;

- **B** représente un groupement choisi parmi les groupements suivants :

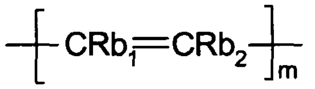

B1

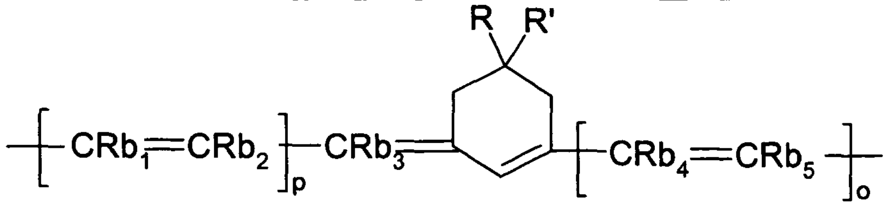

B2

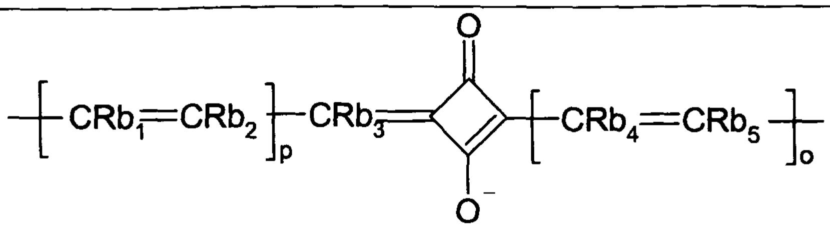

B3

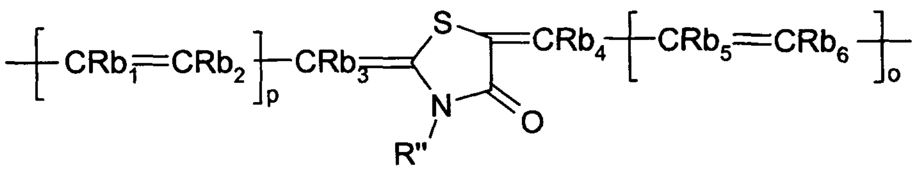

B4

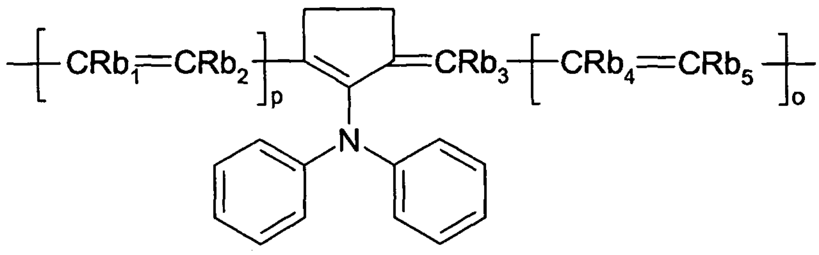

B5

tels que :

- **m** représente un nombre entier de 0 à 3 ;
- **o+p** représente un nombre entier de 0 à 1 ;
- **$Rb_1$, $Rb_2$, $Rb_3$, $Rb_4$, $Rb_5$, $Rb_6$** représentent, indépendamment les uns des autres, un atome d'hydrogène ; un atome d'halogène ; un radical alkyle en $C_1$-$C_3$ ; un radical phényle ;
- **R, R' et R"** représentent indépendamment les uns des autres, un atome d'hydrogène ; un atome d'halogène ou un radical alkyle ;

- **$R_6$** représente un atome d'hydrogène ; un atome d'halogène ; un radical alkyle en $C_1$-$C_3$ ; un radical phényle ;
- **D** représente un groupement choisi parmi les groupements suivants :

| | |
|---|---|
| 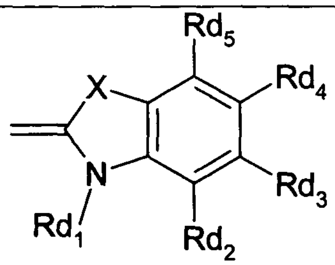 D1 | 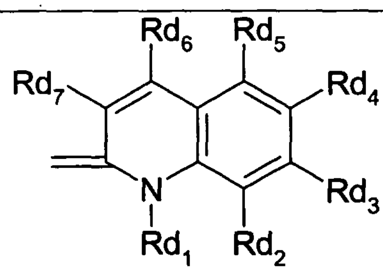 D2 |

| | |
|---|---|
| 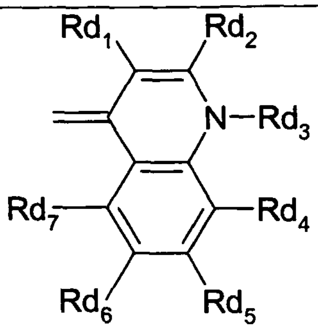 D3 | 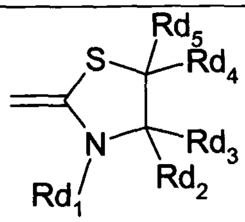 D4 |
| 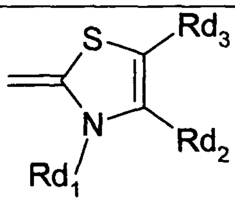 D5 | 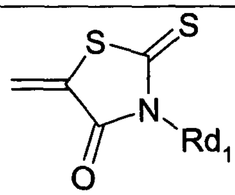 D6 |
| 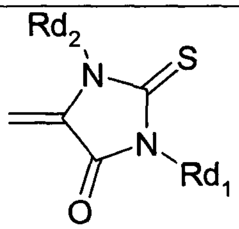 D7 | 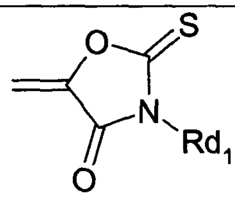 D8 |
| 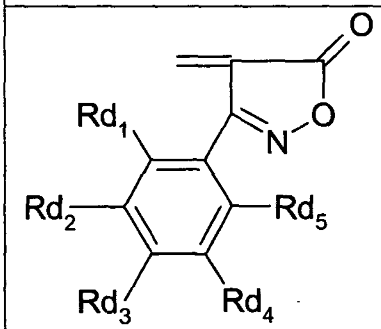 D9 | 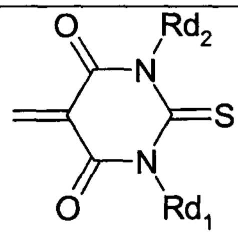 D10 |
| 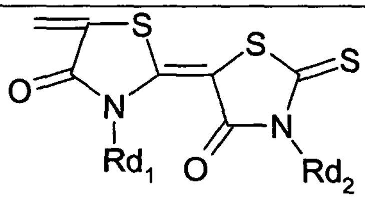 D11 | 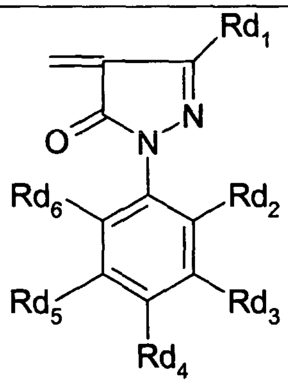 D12 |

| | |
|---|---|
| 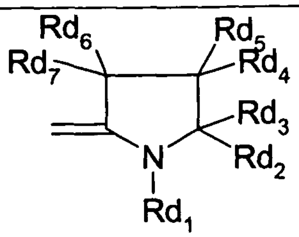 D13 | 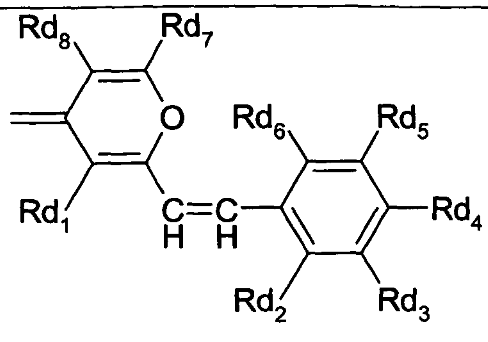 D14 |
| 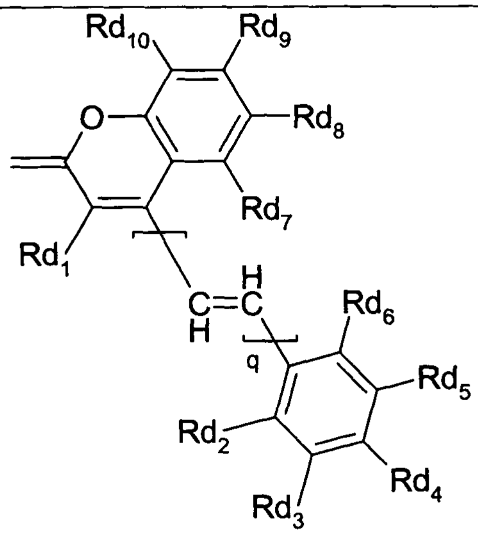 D15 | |

tels que :

- **X** représente un atome d'oxygène, un atome de soufre, un atome de sélénium, un groupement $CR_cR_d$ ou $NR_c$, avec $R_c$ et $R_d$ représentent, indépendamment l'un de l'autre, un atome d'hydrogène ou un radical alkyle ;
- **q** représente un nombre entier égal à 1 ou 2 ;
- **$Rd_1$ à $Rd_{10}$** représentent, indépendamment les uns des autres, un atome d'hydrogène ; un atome d'halogène ; un radical hydroxyle ; un radical alcoxy ; un radical alkyle ; un radical amino ; un radical (alkyl) amino ; un radical di(alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl)amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl) aminoalkyle ; un radical di(hydroxyalkyl)aminoalkyle ; un radical (aminoalkyl)aminoalkyle ; un radical di (aminoalkyl)aminoalkyle ; un radical (alkyl)(hydroxyalkyl)aminoalkyle ; un radical (alkyl)(aminoalkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical carboxy; un radical carboxyalkyle; un radical alkényle ; un groupement sulfonate sous forme acide ou sous forme de sel ; un radical alkyle substitué par un groupement sulfonate sous forme acide ou sous forme de sel ; un radical alkyle substitué par un groupement ammonium quaternaire ; deux groupements adjacents pouvant former ensemble et avec les atomes sur lesquels ils sont fixés un cycle aromatique condensé, substitué ou non.

**2.** Composition selon la revendication 1, dans laquelle $R_1$ à $R_5$ représentent, indépendamment les uns des autres, un atome d'halogène ; un radical hydroxyle ; un radical alcoxy ; un radical alkyle ; un radical amino ; un radical (alkyl)amino ; un radical di(alkyl)amino ; un radical (hydroxyalkyl)amino ; un radical di(hydroxyalkyl)amino ; un radical (aminoalkyl)amino ; un radical di(aminoalkyl)amino ; un radical (alkyl)(hydroxyalkyl)amino ; un radical (alkyl)(aminoalkyl)amino ; un radical (hydroxyalkyl)(aminoalkyl)amino ; un radical hydroxyalkyle ; un radical aminoalkyle ; un radical (alkyl)aminoalkyle ; un radical di(alkyl)aminoalkyle ; un radical (hydroxyalkyl)aminoalkyle ; un radical di(hydroxyalkyl)aminoalkyle; un radical (aminoalkyl)aminoalkyle; un radical di(aminoalkyl)aminoalkyle ; un radical (alkyl)(hydroxyalkyl)aminoalkyle ; un radical (aminoalkyl)(alkyl)amino ; un radical (aminoalkyl)(hydroxyalkyl)aminoalkyle ; un radical phényle éventuellement substitué ; un radical phénylalkyle éventuellement substitué ; un groupement ammonium quaternaire ; un radical alkyle substitué par un groupement ammonium quaternaire ; un radical carboxy ; un radical carboxyalkyle ; un radical alkyle substitué par un groupement sulfonate sous forme acide ou sous forme de sel ; un radical alkylcarbonylalkyle ; deux groupements adjacents pouvant former ensemble et avec les atomes sur lesquels ils sont fixés un cycle aromatique condensé, substitué ou non.

**3.** Composition selon la revendication 1 ou 2, dans laquelle le ou les colorants directs méthiniques contenant un motif benzosélènazolium sont associés à un contre-ion négatif.

**4.** Composition selon la revendication 3, dans laquelle le contre-ion négatif représente un halogénure, un perchlorate, un p-toluène-sulfonate, un m-toluène-sulfonate, un o-toluène-sulfonate, un tétrafluoroborate, un alkyl($C_1$-$C_5$)sulfate ou un sulfonate.

**5.** Composition selon l'une quelconque des revendications 1, 3 et 4, dans laquelle $R_1$ à $R_5$ sont choisis parmi un atome d'hydrogène ; un radical alkyle ; un radical alkyle substitué par un groupement sulfonate sous forme acide ou sous forme de sel.

**6.** Composition selon la revendication 5, dans laquelle $R_1$ à $R_5$ sont choisis parmi un atome d'hydrogène ; un radical méthyle ; un radical éthyle ; un radical n-propyle substitué par un groupement sulfonate sous forme de sel.

**7.** Composition selon l'une quelconque des revendications 1 à 6, dans laquelle n est égal à 1.

**8.** Composition selon l'une quelconque des revendications 1 à 7, dans laquelle A représente un groupement A1.

**9.** Composition selon la revendication 8, dans laquelle $Ra_1$ à $Ra_5$ sont choisis parmi un atome d'hydrogène ; un radical di(alkyl)amino.

**10.** Composition selon la revendication 9, dans laquelle $Ra_1$ à $Ra_5$ sont choisis parmi un atome d'hydrogène ; un radical diéthylamino ; un radical diméthylamino ; un radical méthyléthylamino.

**11.** Composition selon l'une quelconque des revendications 1 à 10, dans laquelle B représente un groupement B1.

**12.** Composition selon la revendication 11, dans laquelle $Rb_1$ et $Rb_2$ sont choisis parmi un atome d'hydrogène ; un radical alkyle.

**13.** Composition selon la revendication 12, dans laquelle $Rb_1$ et $Rb_2$ sont choisis parmi un atome d'hydrogène ; un radical méthyle ; un radical éthyle.

**14.** Composition selon l'une quelconque des revendications 1 à 13, dans laquelle $R_6$ est choisi parmi un atome d'hydrogène.

**15.** Composition selon l'une quelconque des revendications 1 à 14, dans laquelle D représente un groupement D1 ou un groupement D2.

**16.** Composition selon la revendication 15, dans laquelle X représente un atome de sélénium.

**17.** Composition selon la revendication 15 ou 16, dans laquelle $Rd_1$ à $Rd_7$ sont choisis parmi un atome d'hydrogène ; un radical alkyle ; un radical alkyle substitué par un groupement sulfonate sous forme acide ou sous forme de sel.

**18.** Composition selon la revendication 17, dans laquelle $Rd_1$ à $Rd_7$ sont choisis parmi un atome d'hydrogène ; un radical méthyle ; un radical éthyle ; un radical n-propyle substitué par un groupement sulfonate sous forme acide.

**19.** Composition selon l'une quelconque des revendications 1 à 4, dans laquelle le ou les colorants directs méthiniques contenant un motif benzosélènazolium sont choisis parmi les composés de formules (Ia), (IIa) ou (IIb) suivantes :

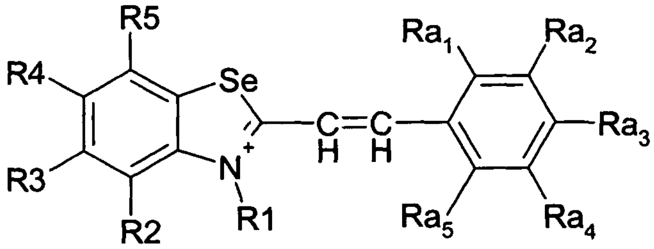

(Ia)

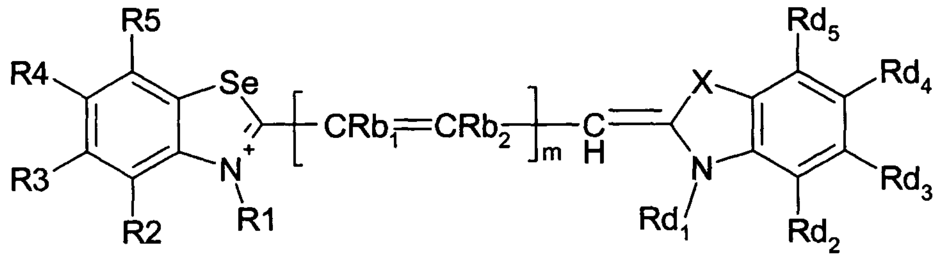

(IIa)

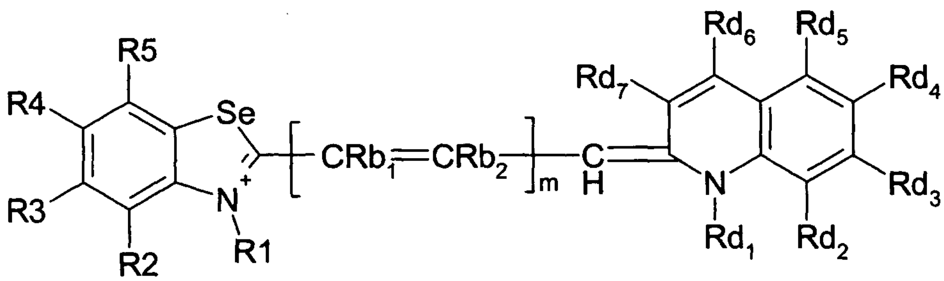

(IIb)

dans lesquelles :

- $\mathbf{R_1}$ à $\mathbf{R_5}$ représentent, indépendamment les uns des autres, un atome d'hydrogène ; un radical alkyle ; un radical alkyle substitué par un groupement sulfonate sous forme acide ou sous forme de sel ;
- $\mathbf{Ra_1}$ à $\mathbf{Ra_5}$ représentent, indépendamment les uns des autres, un atome d'hydrogène ; un radical di(alkyl) amino ;
- m représente un nombre entier de 0 à 3 ;
- $\mathbf{Rb_1}$ et $\mathbf{Rb_2}$ représentent, indépendamment les uns des autres, un atome d'hydrogène ; un radical alkyle en $C_1$-$C_3$ ;
- $\mathbf{Rd_1}$ à $\mathbf{Rd_7}$ représentent, indépendamment les uns des autres, un atome d'hydrogène ; un radical alkyle ; un radical alkyle substitué par un groupement sulfonate sous forme acide ou sous forme de sel ;
- X représente un atome d'oxygène, un atome de soufre, un atome de sélénium, un groupement $CR_cR_d$ ou $NR_c$, avec $R_c$ et $R_d$ représentent, indépendamment l'un de l'autre, un atome d'hydrogène ou un radical alkyle.

**20.** Composition selon la revendication 19, dans laquelle X représente un atome de sélénium.

**21.** Composition selon la revendication 19 ou 20, dans laquelle le ou les colorants directs méthiniques contenant un motif benzosélènazolium sont choisis parmi les colorants suivants :

| | |
|---|---|
| Colorant 1 | 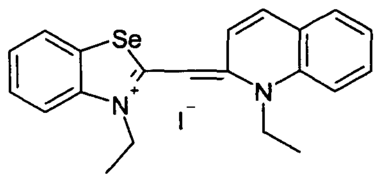 |
| Colorant 2 | 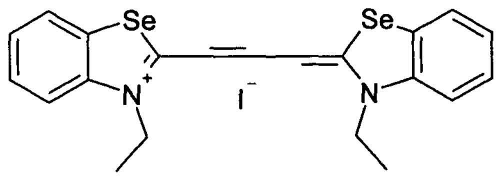 |
| Colorant 3 | 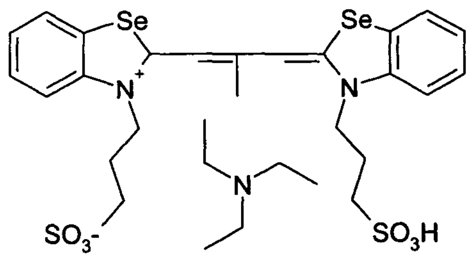 |
| Colorant 4 | 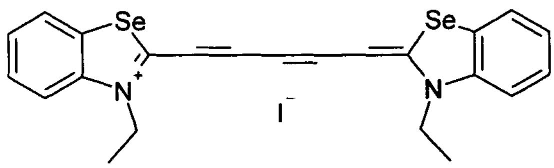 |
| Colorant 5 | 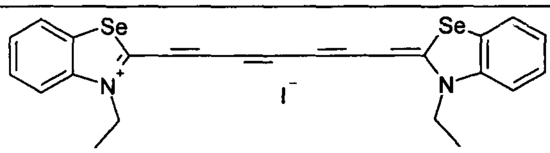 |
| Colorant 6 | 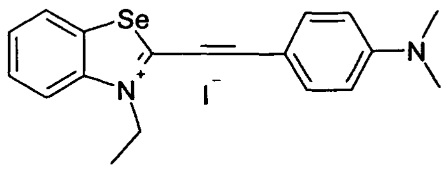 |

| | |
|---|---|
| Colorant 7 | 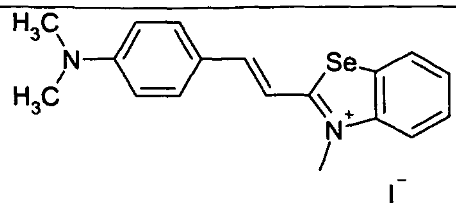 |

**22.** Composition selon l'une quelconque des revendications 1 à 21, dans laquelle le ou les colorants directs méthiniques contenant un motif benzosélènazolium sont chacun présents en quantité comprise entre 0,0001 et 30 % en poids par rapport au poids total de la composition.

**23.** Composition selon l'une quelconque des revendications 1 à 22, comprenant de plus au moins un colorant direct additionnel différent des colorants directs méthiniques contenant un motif benzosélènazolium.

**24.** Composition selon la revendication 23, dans laquelle le ou les colorants directs additionnels sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels, de nature non ionique, anionique ou cationique.

**25.** Composition selon la revendication 23 ou 24, dans laquelle le ou les colorants directs additionnels sont chacun présents en quantité comprise entre 0,0001 et 30 % en poids par rapport au poids total de la composition.

**26.** Composition selon l'une quelconque des revendications 1 à 25, comprenant de plus au moins une base d'oxydation choisie parmi les para-phénylènediamines, les bases doubles, les para-aminophénols, les ortho-aminophénols, les bases hétérocycliques et leurs sels d'addition.

**27.** Composition selon la revendication 26, dans laquelle la ou les bases d'oxydation sont chacune présentes en quantité comprise entre 0,001 et 10 % en poids par rapport au poids total de la composition.

**28.** Composition selon la revendication 26 ou 27, comprenant de plus au moins un coupleur choisi parmi les méta-phénylènediamines, les méta-aminophénols, les méta-diphénols, les coupleurs naphtaléniques, les coupleurs hétérocycliques et leurs sels d'addition.

**29.** Composition selon la revendication 28, dans laquelle le ou les coupleurs sont chacun présents en quantité comprise entre 0,001 et 10 % en poids par rapport au poids total de la composition.

**30.** Procédé de teinture des fibres kératiniques, **caractérisé par le fait que** l'on applique sur les fibres kératiniques une composition telle que définie à l'une quelconque des revendications 1 à 29 pendant un temps de pose suffisant pour obtenir la coloration désirée.

**31.** Utilisation pour la teinture des fibres kératiniques d'un colorant direct méthinique contenant un motif benzosélènazolium.

**32.** Utilisation selon la revendication 31, dans laquelle le ou les colorants directs méthiniques contenant un motif benzosélènazolium sont choisis parmi les composés de formules (I) ou (II) telles que définies à l'une quelconque des revendications 1 à 21.

Figure 1

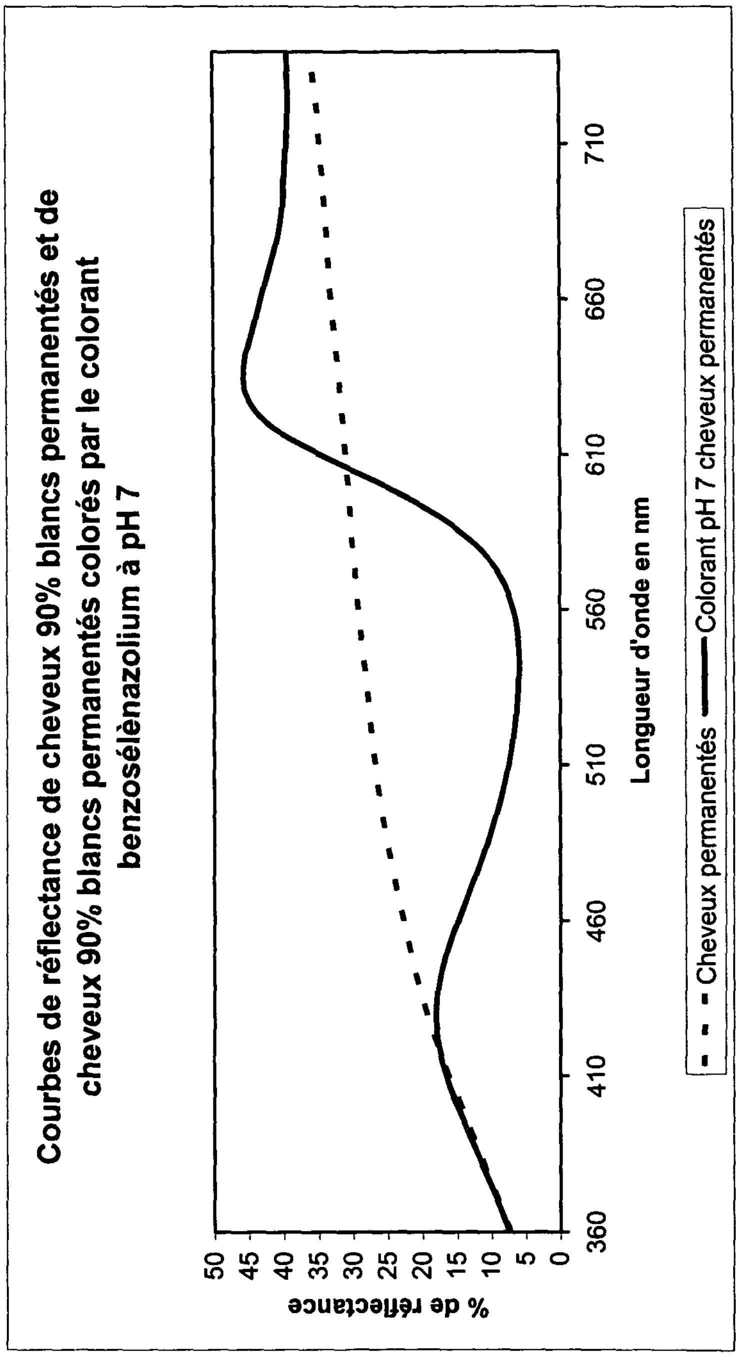
Courbes de réflectance de cheveux 90% blancs permanentés et de cheveux 90% blancs permanentés colorés par le colorant benzosélénazolium à pH 7
% de réflectance
50
45
40
35
30
25
20
15
10
5
0
360
410
460
510
560
610
660
710
Longueur d'onde en nm
Cheveux permanentés
Colorant pH 7 cheveux permanentés

Office européen des brevets

# RAPPORT DE RECHERCHE EUROPEENNE

Numéro de la demande
EP 05 29 1518

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DE 23 38 684 A1 (MATSUSHITA ELECTRIC INDUSTRIAL CO.,LTD) 27 février 1975 (1975-02-27) * revendications 1,8; exemple 7 * | 1-29 | A61Q5/06 A61Q5/10 A61K8/58 A61K8/49 |
| X | DATABASE CA [Online] CHEMICAL ABSTRACTS SERVICE, COLUMBUS, OHIO, US; TOBA, YASUMASA ET AL: "Photopolymerizable composition" XP002360608 extrait de STN Database accession no. 1995:551033 * abrégé * & JP 06 348011 A2 (TOYO INK MFG CO, JAPAN) 22 décembre 1994 (1994-12-22) | 1-29 | |
| X | US 3 554 753 A (ROBERT L. COHEN) 12 janvier 1971 (1971-01-12) * colonne 4, ligne 11 - ligne 24; revendications 1-4 * | 1-29 | |
| | | | DOMAINES TECHNIQUES RECHERCHES (IPC) |
| A | DE 197 32 016 A1 (HENKEL KGAA, 40589 DUESSELDORF, DE) 28 janvier 1999 (1999-01-28) * revendications 1-4 * | 1-32 | A61K |
| A | US 5 474 578 A (CHAN ET AL) 12 décembre 1995 (1995-12-12) * revendication 1; exemple 8 * | 1-32 | |
| | -/-- | | |

Le présent rapport a été établi pour toutes les revendications

3

| Lieu de la recherche | Date d'achèvement de la recherche | Examinateur |
|---|---|---|
| La Haye | 23 décembre 2005 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen des brevets

# RAPPORT DE RECHERCHE EUROPEENNE

Numéro de la demande
EP 05 29 1518

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DATABASE CAPLUS [Online] CHEMICAL ABSTRACTS SERVICE, COLUMBUS, OHIO, US; 22 juillet 1963 (1963-07-22), K. BANNO ET AL: "Semicyanines of the pyrimidylaminovinyl type" XP002326704 extrait de STN Database accession no. 1963:462042 * abrégé * & JP 38 006489 B 20 mai 1963 (1963-05-20) ----- | 1-32 | |
| A | DE 101 14 426 A1 (WELLA AG) 26 septembre 2002 (2002-09-26) * revendications 1,10 * ----- | 1-32 | |
| A | WO 02/22093 A (WELLA AKTIENGESELLSCHAFT; PASQUIER, CECILE; CHARRIERE, VERONIQUE; BRAU) 21 mars 2002 (2002-03-21) * revendications 1,13 * ----- | 1-32 | |
| A | US 5 360 803 A (SHISHIDO ET AL) 1 novembre 1994 (1994-11-01) * colonne 122 - colonne 123; revendications 1,2 * ----- | 1,2 | DOMAINES TECHNIQUES RECHERCHES (IPC) |

Le présent rapport a été établi pour toutes les revendications

3

| Lieu de la recherche | Date d'achèvement de la recherche | Examinateur |
|---|---|---|
| La Haye | 23 décembre 2005 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 1518

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-12-2005

| Document brevet cité au rapport de recherche | Date de publication | Membre(s) de la famille de brevet(s) | Date de publication |
|---|---|---|---|
| DE 2338684 A1 | 27-02-1975 | AUCUN | |
| JP 6348011 A2 | -- | -- | |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 1518

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-12-2005

| Document brevet cité au rapport de recherche | Date de publication | Membre(s) de la famille de brevet(s) | Date de publication |
|---|---|---|---|
| JP 6348011 A2 | | --<br>--<br>--<br>--<br>--<br>-- | |
| US 3554753 A | 12-01-1971 | AUCUN | |
| DE 19732016 A1 | 28-01-1999 | AT 304837 T | 15-10-2005 |
| | | AU 737695 B2 | 30-08-2001 |
| | | AU 8978098 A | 01-03-1999 |
| | | DE 59813067 D1 | 27-10-2005 |
| | | WO 9907334 A1 | 18-02-1999 |
| | | EP 0988022 A1 | 29-03-2000 |
| | | JP 2001513489 T | 04-09-2001 |
| US 5474578 A | 12-12-1995 | AUCUN | |
| JP 38006489 B | -- | --<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>-- | |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 1518

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-12-2005

| Document brevet cité au rapport de recherche | Date de publication | Membre(s) de la famille de brevet(s) | Date de publication |
|---|---|---|---|
| JP 38006489 B | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| DE 10114426 A1 | 26-09-2002 | AUCUN | |
| WO 0222093 A | 21-03-2002 | AU 8192401 A | 26-03-2002 |
| | | BR 0107215 A | 09-07-2002 |
| | | DE 10045600 A1 | 04-04-2002 |
| | | EP 1328244 A1 | 23-07-2003 |
| | | JP 2004508389 T | 18-03-2004 |
| | | US 2003070239 A1 | 17-04-2003 |
| US 5360803 A | 01-11-1994 | AT 144897 T | 15-11-1996 |
| | | AU 649865 B2 | 02-06-1994 |
| | | AU 2097492 A | 25-02-1993 |
| | | CA 2075750 A1 | 14-02-1993 |
| | | CN 1071834 A | 12-05-1993 |
| | | DE 69215035 D1 | 12-12-1996 |
| | | DE 69215035 T2 | 03-04-1997 |
| | | DK 527494 T3 | 25-11-1996 |
| | | EP 0527494 A1 | 17-02-1993 |
| | | ES 2096685 T3 | 16-03-1997 |
| | | FI 923604 A | 14-02-1993 |
| | | GR 3021865 T3 | 31-03-1997 |
| | | HU 64224 A2 | 28-12-1993 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 1518

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-12-2005

| Document brevet cité au rapport de recherche | Date de publication | Membre(s) de la famille de brevet(s) | Date de publication |
|---|---|---|---|
| US 5360803 A | | JP 2567328 B2 | 25-12-1996 |
| | | JP 6172330 A | 21-06-1994 |
| | | NO 923126 A | 15-02-1993 |
| | | ZA 9206004 A | 02-03-1993 |

EPO FORM P0460

Pour tout renseignement concernant cette annexe : voir Journal Officiel de l'Office européen des brevets, No.12/82